# EUROPEAN PATENT SPECIFICATION

(11) **EP 3 344 121 B1**
(45) Date of publication and mention of the grant of the patent: **15.04.2020**
(21) Application number: 16763006.0
(22) Date of filing: 02.09.2016
(51) Int. Cl.: A61B 5/00, G04G 9/00, G04G 99/00, G04G 17/04

(54) **DISPLAY ARRANGEMENT FOR WATCH CASE**
ANZEIGEANORDNUNG FÜR UHRENGEHÄUSE
AGENCEMENT D'AFFICHAGE POUR BOÎTIER DE MONTRE

(30) Priority: 03.09.2015 GB 201515608
(43) Date of publication of application: 11.07.2018
(73) Proprietor: TomTom International B.V., 1011 AC Amsterdam (NL)
(72) Inventor: BAYLEY, Stephen, 1011 AC Amsterdam (NL); MORGAN, David, 1011 AC Amsterdam (NL)
(74) Representative: Siem, Max Yoe Shé
(86) International application number: PCT/EP2016/070737
(87) International publication number: WO 2017/037244

(56) References cited:
- WO-A1-2014/170469
- WO-A2-2007/137264
- WO-A2-2014/135709

## Description

### Field of the Invention

This invention relates to displays for fitness watches and other wearable sensors such as heart rate monitors. The display may be provided as part of a watch case that is mounted, permanently or removably, to a strap so as to be worn on a user's wrist. Illustrative embodiments of the invention relate to fitness watches for monitoring athletic performance, e.g. that can be worn during an exercise activity (running, cycling, swimming, hiking, skiing, weightlifting, etc.), which can track and display information relating to a user's activity levels, such as the heart rate of the user at particular moments during a workout.

### Background of the Invention

Conventional fitness watches typically comprise a liquid crystal display (LCD) module in the watch case. The LCD module may be backlit by one or more light emitting diodes (LEDs). Some fitness watches, e.g. the TomTom® Runner watch, can be used with or without wireless connection to a separate heart rate monitor strap. Other fitness watches, e.g. the TomTom® Cardio watch, include a built-in heart rate monitor, for example in the form of an optical heart rate (OHR) monitor. Users can monitor their training while they exercise by viewing heart rate information displayed on the screen. However, the LEDs used for backlighting can draw a significant amount of power and seriously reduce the time available until the watch battery needs to be recharged. This can affect the usability of the watch, for example for long training sessions or multiple sessions away from a charging facility.

It has been proposed, for example in US 2014/0378844 A1, for a watch to include a communication status indicator comprising an outward facing light source, such as one or more LEDs, in addition to a LCD module, and which is viewable by a user when the watch is in use. The LEDs are in addition to a LCD module of the watch, such that information can be conveyed separately from the LCD display. For instance, the colour of the LED illumination may indicate whether the watch is in communication with another device, e.g. is paired with another device, such as a mobile phone or other computing device over a short-range wireless connection, e.g. Bluetooth. In the MIO® Alpha watch, which comprises an OHR monitor, such an LED arrangement can also be used convey information about whether a user is exercising in a desired heart rate zone. More specially, a green flash is used to indicate that the user is exercising an a target heart rate zone. A red double-flash is used to indicate that the user's heart rate is above the target zone by more than 10 beats per minute (bpm). Finally, a blue double-flash is used to indicate that the user's heart rate is below the target zone by more than 10 bpm. This LED arrangement, however, typically requires a transparent cover over the LEDs and this means that the underlying LEDs are visible even when not illuminated, which can cause confusion. Especially when viewing the watch display in daylight conditions, it can be difficult for a user to discern whether such LEDs are illuminated or not.

It is desired, in at least embodiments of the present invention, to provide an improved display arrangement for a watch case, and preferably for a fitness watch case.

### Summary of the Invention

In accordance with a first aspect of the present invention there is provided a display arrangement for a watch case comprising: a display module; a substantially transparent cover extending over the display module, the cover comprising a layer forming a substantially opaque area outside the display module; and one or more light emitting devices arranged below the opaque area, wherein the layer comprises a plurality of discontinuities that allows light to be transmitted through the opaque area from the one or more light emitting devices.

According to the present invention, it is possible to hide the one or more light emitting devices from view by positioning them below the opaque area of the cover. However it is still possible for a user to see light from the one or more light emitting devices because the plurality of discontinuities allows light to be transmitted through the opaque area when the device(s) are in use. This provides the benefit of an illuminated indicator that is separate to the display module and, furthermore, the illumination of such an indicator being clearly discernible regardless of the ambient lighting conditions. Even in daylight, a user can readily see when the opaque area is dark and when there is light being transmitted through the opaque area against a dark background.

The display module can be of any suitable form, such as a liquid crystal display (LCD), a light emitting diode (LED) display, a polymer light emitting diode (PLED) display, an organic light emitting diode (OLED) display, etc. In preferred embodiments, however, the display modules is one that require a backlight in order to produce a visible image, such as a LCD.

The substantially transparent cover can also be of any suitable form, such as glass, sapphire crystal, etc. As will be appreciated, the cover is typically used to protect the display module and other components of the watch case, and thus preferably has an external surface and an internal surface; the internal surface facing the display module. As will be appreciated, the layer is typically on the internal surface of the cover. The display module can be in contact with the internal surface of the cover, although typically the display module is spaced from the cover, such that there is a gap between the two components.

The substantially opaque area formed by the layer on the cover preferably forms a substantially opaque frame surrounding the display module. This opaque frame can conveniently hide the physical frame or edges of the display module. The one or more light emitting devices may therefore be aligned with the opaque frame of the cover, rather than requiring a separate cover. The display arrangement of the watch case is preferably defined by a single cover that extends over the display module, e.g. LCD, and the one or more light emitting devices. This can simplify the construction of the case.

The plurality of discontinuities may take the form of lines (e.g. straight, curved, zigzag, etc.) or holes (round, square, rectangular, polygonal, etc.) in the layer of the substantially transparent cover. The layer preferably comprises a printed layer. The discontinuities may be formed at the same time as the printed layer, for example using a printing mask, or the discontinuities may be formed after the printed layer, for example by removing some of the printed layer. In a preferred set of embodiments, the plurality of discontinuities comprises holes in the layer that are between 0.1 and 0.5 mm wide, preferably about 0.2 mm wide. The plurality of discontinuities may be formed in a pattern. The pattern may be random but is preferably substantially uniform.

The one or more light emitting (or illumination) devices may be arranged directly below the opaque area such that light is incident on the plurality of discontinuities. However, this may be difficult to achieve when the display module is positioned beneath the cover, especially when the opaque area is also used to hide the edges of the display module. It is therefore preferable that the one or more light emitting devices are arranged below the display module and/or to one side of the display module. This means that light emitted by the one or more illumination devices may dissipate before it reaches the plurality of discontinuities in the printed layer. In a preferred set of embodiments the display further comprises at least one light guide arranged below the cover to guide light from the one or more illumination devices to the plurality of discontinuities in the layer of the cover. Using a light guide can ensure that there is no substantial loss of amplitude before light reaches the plurality of discontinuities where it is transmitted through the opaque area. Using a light guide can also provide more flexibility in the position of the one or more illumination devices below the cover, for example allowing the one or more illumination devices to be accommodated where there is space around and/or beneath the display module.

It is mentioned above that the opaque area formed by the layer is preferably arranged above the display module. Accordingly the plurality of discontinuities in the layer may also be positioned above the display module. In a preferred set of embodiments, at least one light guide is arranged such that light is guided so as to pass through the display module before reaching the plurality of discontinuities in the layer of the cover. For example, a transparent plastic light guide may extend from the one or more illumination devices to direct light to the underside of the display module.

The one or more illumination devices could be arranged below the display module, but typically the display module is mounted on a circuit board and there may not be space to accommodate the illumination devices therebetween. In a preferred set of embodiments the one or more illumination devices are arranged next to the display module below the cover. Preferably the one or more illumination devices and the display module are mounted next to one another on a shared circuit board. This provides a compact arrangement for the display of the watch case. The cover may simply extend over and beyond the display module so that the one or more illumination devices are positioned below the opaque area formed by the layer.

The one or more illumination devices may comprise any suitable light emitting device. In a preferred set of embodiments the one or more illumination devices comprise LEDs. It will be appreciated that these LEDs are separate to any LEDs that may be incorporated in the display module, e.g. LCD, itself for backlighting purposes.

It has been recognised that the colour of light emitted by the one or more illumination devices can be used to convey information to a user of the display. In a preferred set of embodiments the one or more illumination devices can emit a plurality of different colours of light; preferably at least three different colours (e.g. red, green, blue), and further preferably at least five different colours of light (red, green blue, purple and turquoise). The one or more illumination devices can comprise a single illumination device that is capable of emitting light in different colours, or can comprise a plurality of illumination devices that are each arranged to emit light of a single colour.

A display arrangement according to embodiments of the present invention may be incorporated into a fitness watch or other wearable sensor, for example a heart rate monitor. In a preferred set of embodiments there is provided a watch case comprising the display arrangement as described above. The case may house one or more other components that interact with the display. Preferably the watch case comprises one or more components configured to measure and/or receive heart rate information. In a set of embodiments the watch case further comprises a transceiver and processor arranged to receive heart rate information from an external heart rate monitor. The watch case may, for example, be paired via Bluetooth® with a heart rate monitor mounted on a chest strap. In another set of embodiments the watch case further comprises an optical heart rate (OHR) monitor arranged to measure heart rate information. When the watch case is worn on a user's wrist, the OHR monitor can measure heart rate using a built-in LED arranged to emit light into the skin, where it is partially absorbed by the underlying blood vessels, and light reflected back through the skin is sensed by a photodetector and the signals processed to determine heart rate information.

In addition, or alternatively, the watch case for a fitness watch may comprise one or more components configured to measure and/or receive information relating to other parameters of relevance to a user's activity. The watch case may include location determining means, e.g. a global navigation satellite system (GNSS) receiver, such as GPS and/or GLONASS. In a set of embodiments the watch case further comprises a GPS receiver and processor arranged to measure one or more parameters relating to a user's activity. Such parameters may include, for example, one or more of: speed, acceleration, cadence, distance, time (e.g. in relation to a given physical activity such as running, cycling, etc.). In addition, or alternatively, the watch case may further comprise one or more sensors including: a gyroscope, an altimeter, a pressure sensor (e.g. diving depth gauge), an electronic compass, and a wireless communication hub (for example capable of receiving signals from one or more body-worn sensors). Such sensors may be used to measure one or more parameters relating to a user's physical activity.

In at least some embodiments, the watch case may be configured to determine heart rate information indirectly from the one or more parameters measured by the GPS receiver and/or other sensor(s). For example, the watch module may comprise a processor including a memory that stores a look-up table equating the measured parameters (e.g. speed, altitude, etc) with a common heart rate for such a level of physical activity.

Regardless of whether heart rate information is measured directly by the watch module, received from an external sensor and/or determined indirectly from one or more other measured parameters, the one or more illumination devices may advantageously be used to indicate this information to a user. In a preferred set of embodiments the one or more illumination devices are controlled so as to flash at a frequency that is dependent on heart rate information. For example, the one or more illumination devices may be controlled so as to flash at a frequency that approximates the user's heart rate. This means that a user can quickly and easily see their current heart rate simply by glancing at the flashing illumination. Furthermore, the applicant has recognised that this enables heart rate information to be displayed without the display module, e.g. LCD, being active, thereby saving battery power for the watch module.

This is considered novel and inventive in its own right, and thus when viewed from a second aspect the present invention provides a watch case comprising: a display module; one or more light emitting devices external to the display module; and a processor configured to receive heart rate information, wherein the one or more light emitting devices are controlled by the processor so as to flash at a frequency that is dependent on the heart rate information.

Preferably the one or more light emitting (or illumination) devices are controlled so as to flash at a frequency that approximates a user's heart rate. As is described above, such a watch case may receive heart rate information from an external heart rate monitor or, preferably, the watch module comprises an optical heart rate (OHR) monitor arranged to measure the heart rate information. The heart rate information can also be estimated indirectly from one or more other measured parameters, e.g. as described above.

In addition, or alternatively, the one or more illumination devices may be controlled so that the colour of emitted light corresponds to a predetermined heart rate zone. The predetermined heart rate zone may be based on heart rate information this is directly measured or indirectly determined, as described above.

The present invention according to this second aspect, or any embodiments thereof, may include any or all of the features described in relation to the first aspect of the invention to the extent that they are not mutually inconsistent. For example, the fitness watch case may comprise a substantially transparent cover extending at least over the display module, and preferably over the display module and the one or more light emitting devices. Similarly, the cover may comprise a layer forming a substantially opaque area outside the display module, and wherein the layer comprises a plurality of discontinuities that allows light to be transmitted through the opaque area from the one or more light emitting devices.

There will now be described some general features of the watch case that may be combined with one or more of the embodiments outlined above.

The watch case is preferably configured as a single integral module, and which is preferably a sealed module being water resistant to allow the module to be used for wet weather outdoor exercise and for swimming.

In various examples the watch case may comprise a user interface including an input device, e.g. in the form of one or more push buttons. In a preferred set of embodiments the casing comprises a display housing that houses the display arrangement (also referred to herein as the "display") and an input device for controlling the display arrangement, wherein the input device is spaced apart from the display housing. Accordingly the input means is preferably spaced apart from the display housing, for example in a longitudinal direction of the strap when the watch case is so mounted. The display arrangement, and in this case preferably the display module, may be configured to display alphanumeric characters or icons such that upper parts of the characters or icons are arranged towards a first side of the display housing and the lower parts of the characters or icons are arranged towards a second, opposite side of the display housing. The input device is preferably spaced apart from the display housing in a direction from said first side to said second side. This configuration is useful when a user wears the display housing on the back of the wrist, as the user is easily able to view the display whilst controlling the device via the input device that is spaced apart from the display. Less preferably, the input device may be spaced apart from the display housing in a direction from said second side to said first side of the display housing. This configuration may be useful, for example, when the device is strapped to the handle bars of a bicycle or strapped to another vehicle, as the display can be directed towards the user whilst the user has easy access to the input device from above the module.

The input device is preferably configured to control the display module and associated electrical components in use. For example, the input device may be configured for navigating through a menu displayed on the display. For example, the input device may control the functioning of the OHR sensor, where one is included in the watch module. The input device is therefore electrically connected to electronic components in the display housing. For example, a ribbon lead may extend between the display housing and the input device.

The input device preferably has a substantially planar surface arranged substantially parallel to and above an upper surface of the module. The input device is preferably configured to detect the movement of a user's finger across the substantially planar surface so as to provide an input to control the module, e.g. for navigating a menu displayed on the display.

The input device may therefore comprise a touchpad (or trackpad) utilising, for example, capacitive sensing to conductance sensing to translate the motion of a user's finger into an input to control the watch module. The touchpad may comprise a one-dimensional touchpad, and which is capable of sensing motion along a single axis, e.g. left-right or up-down. In other more preferred embodiments, the touchpad may comprise a two-dimensional touchpad, and which is capable of sensing motion in any direction, or at least left-right and up-down, on the plane defined by the substantially planar surface of the input means. In other, albeit less preferred embodiments, the input device may comprise a pointing stick (or trackpad) that senses the force applied by a user's finger, e.g. by using a pair of resistive strain gauges, and translates it into an input to control the watch module.

Alternatively, the input device may comprise a two-way button having a continuous pressing surface and two actuators, the button being configured such that when a first portion of the pressing surface is depressed a first of said actuators is actuated so as to provide a first input to control the module, and when a second portion of the pressing surface is depressed a second of said actuators is actuated so as to provide a second input to control the module.

Alternatively, the input device may comprise a four-way button having a continuous pressing surface and four actuators, the button being configured such that when a first portion of the pressing surface is depressed a first of said actuators is actuated so as to provide a first input to control the module, when a second portion of the pressing surface is depressed a second of said actuators is actuated so as to provide a second input to control the module, when a third portion of the pressing surface is depressed a third of said actuators is actuated so as to provide a third input to control the module, and when a fourth portion of the pressing surface is depressed a fourth of said actuators is actuated so as to provide a fourth input to control the module. The pressing surface described herein is preferably a substantially planar surface parallel to and above a portion of a lower surface that contacts a user's limb in use. It is also contemplated that the input device may comprise any one or more mechanically actuated buttons or non-mechanically actuated buttons, such as virtual buttons on a touch-sensitive user interface, as desired.

The input device is preferably additionally, or alternatively, configured to be operated by being pressed in a direction that is substantially perpendicular to its substantially planar surface, in a direction from the upper surface towards the lower surface. This enables the user to use a single finger to operate the input device. The user does not need to use a second finger of the same hand to counter-balance the pressing of the input device, because the input device is arranged such that it is pressed against the wrist of the user wearing the watch module.

In preferred embodiments in which the input device is configured to both detect the movement of a user's finger across the substantially planar surface and be pressed against the limb of the user, e.g. where the input device comprises a depressible touch pad, the detected motion of the user's finger is used to navigate a menu for identifying a function to be selected, and the depression of the input device is used to select the identified function.

In addition, or alternatively, the display is preferably substantially planar, arranged in a first plane, and the input device preferably has a substantially planar (pressing) surface arranged in a second plane, wherein the first and second planes are at angles to each other. The dihedral (or torsion) angle between the two planes is preferably less the 90 degrees, and preferably between 20 and 70 degrees. In other words, the planes are imaginary intersecting planes and the sides of the planes facing the user's arm or wrist in use define an angle between them at the intersection, wherein the angle is preferably greater than 90 degrees and less than 180 degree. By providing the surfaces at an angle to each other, the user is enabled a good viewing angle of the display whilst operating the input device, when the watch module is mounted to a user's wrist in use. As the input device is spaced away from the display housing, and hence away from the back of the user's wrist and around the side of the wrist in use, said angle also enables the input device to be orientated such that when it is pressed it is pressed against the user's wrist such that the user's wrist provides the counter-force necessary to balance the pressing force. The input device is therefore able to be operated with a single finger and without needing a second finger on the same hand to counter-balance the pressing force as in conventional watches having buttons around the periphery of the display housing.

In addition, or alternatively, the display housing is preferably physically connected to the input device by a connecting portion, wherein the connecting portion is curved or angled along the direction from the display housing to the input device. The connecting portion may be curved or angled such that when the display housing is arranged on the back of a user's wrist in use, the connecting portion curves or otherwise extends around the wrist such that the input device is located on the side of the user's wrist. The watch module is preferably configured such that the input device is located on the medial side of the user's wrist when the display housing is located on the back of the wrist, the medial side being the side facing the user's body when the back of the hand is facing vertically upwards. In other less preferred embodiments a wrist strap may form said connecting portion that connects the display housing portion and the input means. The strap may be flexible or formed from one or more pivotable sections so as to flex or pivot to form the curved or angled connecting portion.

The watch case preferably comprises a processor configured to control the display. The display may visually display heart rate (HR) data such as one or more of: current HR (bpm), average HR (bpm), maximum HR, minimum HR; current HR zone; a graphical representation of HR changes over time; and a graphical representation of the proportion of time spent in each of a plurality of HR zones over time. In addition, or alternatively, the watch module may comprise an audio output, e.g. a beeper, and/or a haptic output, e.g. a vibrator, to alert a user to changes in the HR data.

In addition, or alternatively, a or the processor may be connected to means for tracking the location of a user as he or she moves from one location to another, e.g. by using information received from global navigation satellite signals, or by accessing and receiving information from WiFi access points or cellular communication networks. In preferred embodiments the watch module comprises a global navigation satellite system (GNSS) receiver, such as a GPS and/or GLONASS receiver, for receiving satellite signals indicating the position, and optionally speed, of the receiver (and thus user) at a particular point in time, and which receives updated information at regular intervals. As will be appreciated, this adds the functionality of tracking the location of the user as he or she moves from one location to another. The GNSS receiver may comprise an antenna, e.g. in the form of a patch antenna, for use in determining the location and movements of the user.

The watch case may comprise one or more of: a pressure sensor for measuring atmospheric pressure (for use in determining altitude and/or depth); a pulse sensor; a vibration device for indicating alerts to a user; an accelerometer; an electronic compass; or a wireless communications device, such as a Bluetooth module (e.g. capable of using the Bluetooth Low Energy (BLE) protocol). In embodiments where the watch case comprises a wireless communications device, this may be arranged to receive data from other sensors, such as a foot pod sensor or a speed/cadence sensor. As mentioned above, the wireless communications device may be arranged to communicate with an external heart rate monitor, for example a monitor mounted on a chest strap worn by the user. In addition, or alternatively, the wireless communications device may be arranged to transmit data to one or more external devices (e.g. a mobile phone device).

The watch case may comprise one or more electrical connectors for electrically connecting to a dock or cable for charging the battery and/or for transferring data to or from the processor. It is contemplated that any known electrical connector may be employed. In preferred embodiments, however, the one or more electrical connectors comprise electrical contacts, which may be flat and arranged substantially in line with, or recessed in, the lower surface of the case (e.g. for contacting with corresponding pogo pins in a docking system). The electrical contacts may be located in any portion of the lower surface of the case as desired, although in preferred embodiments the electrical contacts are located in the lower surface under the input device, e.g. distal from the display housing. This allows the user to see the display when the module is positioned in a docking system.

The present invention also extends to a watch case comprising a display arrangement according to the first aspect, or any embodiments thereof, and further extends to a fitness watch comprising a watch case according to any of the aspects and embodiments as described above.

In at least some embodiments, the watch case (or module) may be permanently mounted by a wrist strap. For example, the watch case may be integrated with the strap. However, in various embodiments of the present invention, the watch module is preferably removably mounted to a wrist strap. For example, the strap may comprise a central mount to which the watch module is removably connected. This can allow the watch module to be repeatedly engaged and disengaged from the strap, for example so that a user can dock the module to allow for the transfer of power and/or data, e.g. using a docking station connected to a computer. In addition, or alternatively, the same strap may be used interchangeably to mount other watch modules or different sensors.

The present invention in accordance with any of its further aspects or embodiments may include any of the features described in reference to other aspects or embodiments of the invention to the extent it is not mutually inconsistent therewith.

Advantages of these embodiments are set out hereafter, and further details and features of each of these embodiments are defined in the accompanying dependent claims and elsewhere in the following detailed description.

### Brief Description of the Drawings

Various aspects of the teachings of the present invention, and arrangements embodying those teachings, will hereafter be described by way of illustrative example with reference to the accompanying drawings, in which:
Figure 1 shows a perspective view of a watch case (or module);
Figure 2 shows the watch module of Figure 1 as viewed from the underside;
Figure 3a shows a side sectional view of the watch module of Figure 1;
Figure 3b shows a close-up of the side sectional view of Fig. 3a;
Figure 4 is a schematic illustration of electronic components of a fitness watch according to a preferred embodiment;
Figure 5 is a schematic illustration of the manner in which a fitness watch may receive information over a wireless communication channel; and
Figure 6 shows a close-up of the top of the watch module of Figure 1.

### Detailed Description of the Preferred Embodiments

Preferred embodiments of the present invention will now be described with particular reference to a fitness or sports watch having access to Global Positioning System (GPS) data. Fitness or sports watches of the type described are often worn by athletes to help them during their runs or workouts, e.g. by monitoring the speed and distance of the user and providing this information to the user. It will be appreciated, however, that the device could be arranged to be carried by a user or connected or "docked" in a known manner to a vehicle such as a bicycle, kayak, or the like.

In general, GPS is a satellite-radio based navigation system capable of determining continuous position, velocity, time, and in some instances direction information for an unlimited number of users. Formerly known as NAVSTAR, the GPS incorporates a plurality of satellites which orbit the earth in extremely precise orbits. Based on these precise orbits, GPS satellites can relay their location to any number of receiving units.

The GPS system is implemented when a device, specially equipped to receive GPS data, begins scanning radio frequencies for GPS satellite signals. Upon receiving a radio signal from a GPS satellite, the device determines the precise location of that satellite via one of a plurality of different conventional methods. The device will continue scanning, in most instances, for signals until it has acquired at least three different satellite signals (noting that position is not normally, but can be determined, with only two signals using other triangulation techniques). Implementing geometric triangulation, the receiver utilizes the three known positions to determine its own two-dimensional position relative to the satellites. This can be done in a known manner. Additionally, acquiring a fourth satellite signal will allow the receiving device to calculate its three dimensional position by the same geometrical calculation in a known manner. The position and velocity data can be updated in real time on a continuous basis by an unlimited number of users.

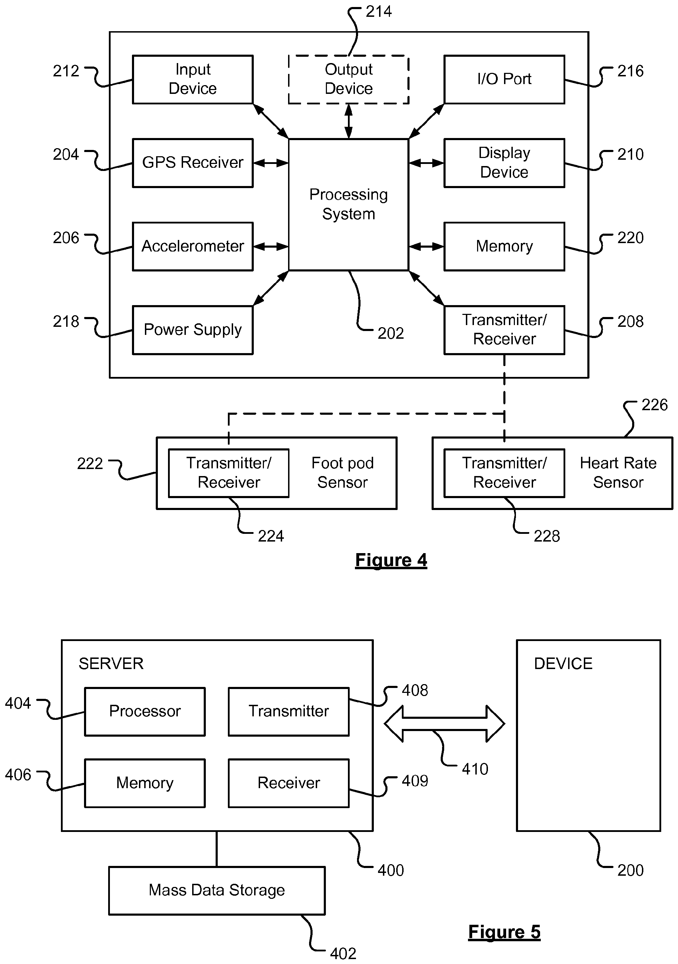

Figure 4 is an illustrative representation of electronic components of a sports watch 200 according to a preferred embodiment of the present invention, in block component format. It should be noted that the block diagram of the device 200 is not inclusive of all components of the device, but is only representative of many example components.

The device 200 includes a processor 202 connected to an input device 212, such as a depressible touchpad (or trackpad), and a display screen 210, such as an LCD display. The device 200 can further include an output device arranged to provide audible information to a user, such as alerts that a certain speed has been reached or a certain distance has been travelled.

Figure 4 further illustrates an operative connection between the processor 202 and a GPS antenna/receiver 204. Although the antenna and receiver are combined schematically for illustration, the antenna and receiver may be separately located components. The antenna may be of any suitable form, but in preferred embodiments is a GPS patch antenna.

The device 200 further includes an accelerometer 206, which can be a 3-axis accelerometer arranged to detect accelerations of the user in x, y and z directions. The accelerometer may act as a pedometer for use when/if there is a loss of GPS reception, and/or may act to detect stroke rate when the fitness watch is being used during swimming. Although the accelerometer is shown to be located within the device, the accelerometer may also be an external sensor worn or carried by the user, and which transmits data to the device 200 via the transmitter/receiver 208.

The device may also receive data from other sensors, such as a foot pod sensor 222 or a heart rate sensor 226. The foot pod sensor may, for example, be a piezoelectric or micro-electro-mechanical systems (MEMS) accelerometer that is located in or on the sole of the user's shoe. Each external sensor is provided with a transmitter and receiver, 224 and 228 respectively, which can be used to send or receive data to the device 200 via the transmitter/receiver 208.

The processor 202 is operatively coupled to a memory 220. The memory resource 220 may comprise, for example, a volatile memory, such as a Random Access Memory (RAM), and/or a non-volatile memory, for example a digital memory, such as a flash memory. The memory resource 220 may be removable. As discussed in more detail below, the memory resource 220 is also operatively coupled to the GPS receiver 204, the accelerometer 206 and the transmitter/receiver 208 for storing data obtained from these sensors and devices.

Further, it will be understood by one of ordinary skill in the art that the electronic components shown in Figure 4 are powered by a power source 218 in a conventional manner. The power source 218 may be a rechargeable battery.

The device 200 further includes an input/output (I/O) device 216, such as a plurality of electrical contacts or a USB connector. The I/O device 216 is operatively coupled to the processor, and also at least to the memory 220 and power supply 218. The I/O device 216 is used, for example, to: update firmware of processor 220, sensors, etc; transfer data stored on the memory 220 to an external computing resource, such as a personal computer or a remote server; and recharge the power supply 218 of the device 200. Data could, in other embodiments, also be sent or received by the device 200 over the air using any suitable mobile telecommunication means.

As will be understood by one of ordinary skill in the art, different configurations of the components shown in Figure 4 are considered to be within the scope of the present application. For example, the components shown in Figure 4 may be in communication with one another via wired and/or wireless connections and the like.

In Figure 5 the watch 200 is depicted as being in communication with a server 400 via a generic communications channel 410 that can be implemented by any number of different arrangements. The server 400 and device 200 can communicate when a connection is established between the server 400 and the watch 200 (noting that such a connection can be a data connection via mobile device, a direct connection via personal computer via the internet, etc.).

The server 400 includes, in addition to other components which may not be illustrated, a processor 404 operatively connected to a memory 406 and further operatively connected, via a wired or wireless connection, to a mass data storage device 402. The processor 404 is further operatively connected to transmitter 408 and receiver 409, to transmit and send information to and from device 200 via communications channel 410. The signals sent and received may include data, communication, and/or other propagated signals. The functions of transmitter 408 and receiver 409 may be combined into a signal transceiver.

The communication channel 410 is not limited to a particular communication technology. Additionally, the communication channel 410 is not limited to a single communication technology; that is, the channel 410 may include several communication links that use a variety of technology. For example, the communication channel 410 can be adapted to provide a path for electrical, optical, and/or electromagnetic communications, etc. As such, the communication channel 410 includes, but is not limited to, one or a combination of the following: electric circuits, electrical conductors such as wires and coaxial cables, fibre optic cables, converters, radio-frequency (RF) waves, the atmosphere, empty space, etc. Furthermore, the communication channel 410 can include intermediate devices such as routers, repeaters, buffers, transmitters, and receivers, for example.

In one illustrative arrangement, the communication channel 410 includes telephone and computer networks. Furthermore, the communication channel 410 may be capable of accommodating wireless communication such as radio frequency, microwave frequency, infrared communication, etc. Additionally, the communication channel 410 can accommodate satellite communication.

The server 400 may be a remote server accessible by the watch 200 via a wireless channel. The server 400 may include a network server located on a local area network (LAN), wide area network (WAN), virtual private network (VPN), etc.

The server 400 may include a personal computer such as a desktop or laptop computer, and the communication channel 410 may be a cable connected between the personal computer and the watch 200. Alternatively, a personal computer may be connected between the watch 200 and the server 400 to establish an internet connection between the server 400 and the watch 200. Alternatively, a mobile telephone or other handheld device may establish a wireless connection to the internet, for connecting the watch 200 to the server 400 via the internet.

The server 400 is further connected to (or includes) a mass storage device 402. The mass storage device 402 contains a store of at least digital map information. This digital map information can be used, together with data from the device, such as time-stamped location data obtained from the GPS receiver 204 and data indicative of motion of the wearer obtained from the accelerometer 206, footpad sensor 222, etc, to determine a route travelled by the wearer of the device 200, which can then be viewed by the wearer.

As will be appreciated, the watch 200 is designed to be worn by a runner or other athlete as they undertake a run or other similar type of workout. The various sensors within the watch 200, such as the GPS receiver 204 and the accelerometer 206, collect data associated with this run, such as the distance travelled, current speed, etc, and display this data to the wearer using the display screen 210.

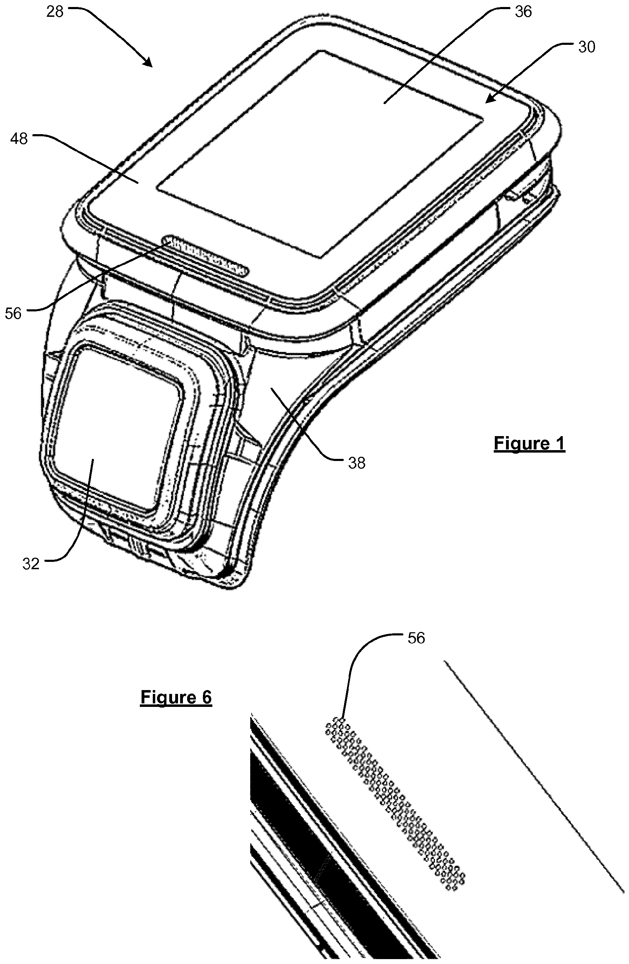

Figure 1 shows a perspective view of a watch case (or module) 28 according to a preferred embodiment of the present invention, which is in the form of a module that can be inserted into a plurality of different docking solutions. The watch module 28 comprising a display housing 30 and an input device 32 that is spaced apart from the display housing 30. The display housing 30 is of substantially parallelepiped construction and has a substantially planar display 36 for displaying information to the user. The display 36 is framed by an opaque area 48. The input device portion 32 is connected to the display housing 30 by a connecting portion 38. The connecting portion 38 can be seen as a curved flange that extends away from the display housing 30. The curved flange 38 extends away from the display housing 30 such that it curves around a user's wrist when the watch module is mounted to a wrist strap (not shown). The input device 32 is located so as to be arranged on the side of the user's wrist in use. The input device 32 has a substantially planar pressing surface for the user to interact with the watch module 28. The user can thereby press the pressing surface in a direction perpendicular to the pressing surface so as to control the watch module 28, e.g. to select desired functions within the menu system of the watch. In this example the input device 32 takes the form of a four-way button.

The location of the input device 32 being arranged on the curved flange 38 such that it sits against the side of the user's wrist in use has a number of important advantages. For example, this enables the user to interact with the watch module 28 using only a single finger. More specifically, the user is able to push the pressing surface of the input device 32 with one finger because the user pushes the surface into the user's wrist around which the watch 28 is strapped. This is in contrast to conventional watches wherein buttons are arranged around the peripheral edges of the watch and the user must press the button with on finger and use a thumb on the other edge of the watch to counter-balance the pressing force. As seen in Figure 1, for example, the plane defined by the substantially planar display 36 is arranged at an angle to the plane defined by the input device 32, the dihedral angle between the two planes being less than 90 degrees, and typically between 20 and 70 degrees.

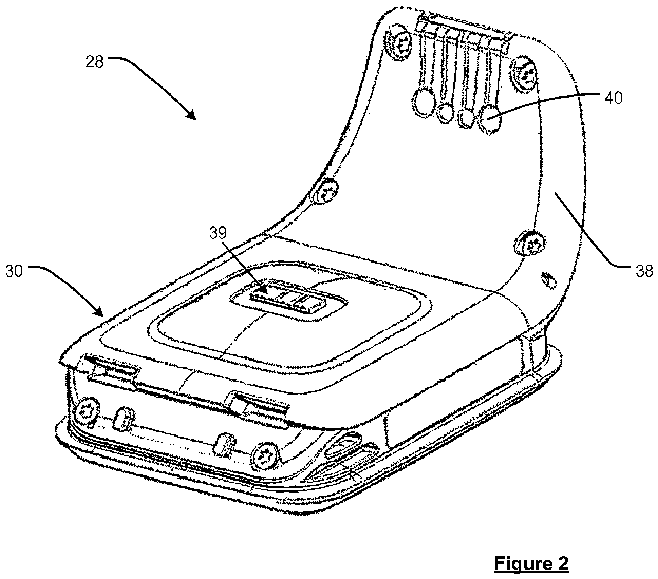

Figure 2 shows a perspective view of the watch module 28 from the underside. The curved flange 38 that extends from the display housing 30 has electrical connectors 40 arranged at a distal end thereof. These electrical connectors 40 may be used in order to electrically connect the watch module 28 to a dock in order to recharge a battery within the watch module 28 and/or to extract data from or input data to the watch module 28. As the electrical connectors 40 are arranged at a distal end of the module 28, they are optimally arranged to be inserted into the slot of a docking device that has docking electrical connections for engaging the electrical connectors 40. Alternatively, the electrical connectors 40 can be arranged to connect with a cable, e.g. a USB cable.

The underside of the display housing 30 may include an optical heart rate (OHR) sensing unit 39 protruding therefrom. The OHR sensing unit 39 comprises a pair of light emitting diodes (e.g. green LEDs) arranged either side of a photodetector, although more LEDs that emit light at different wavelengths could also be included. When the watch module 28 is mounted to a wrist by a strap, the sensing unit 39 sits on the back of the wrist in contact with the skin. The OHR sensing unit 39 may be operatively connected to a processor in the watch module 28 that can process data signals relating to pulse and heart rate. The processor is typically connected to a memory and a power supply, e.g. battery. The battery may be recharged when the module 28 is docked using its I/O port, for example in the form of the electrical connectors 40 described above. The same electrical connectors 40 may also be used to transfer data to/from the processor. In addition to the I/O port, the watch module 28 may include a wireless communications interface, such as a Bluetooth transceiver, that enables the watch module 28 to wirelessly communicate with one or more other devices to receive additional data. For example, other devices that are body-worn (e.g. an external heart rate monitor) or mounted nearby during exercise (e.g. mounted to a bike during cycling activities) may pair with the watch module 28 to transmit additional data. The sensor module's user interface may allow a user to view such additional data on the wrist-mounted device. In some embodiments the watch module 28 could take the form of a fitness watch module, in particular a GNSS, e.g. GPS, watch module.

The watch module's user interface includes the display 36 and the input device 32 already described above. Of course other user interface components may be provided instead, or as well as, those seen in the figures. Further features of a watch module 28 as seen in Figures 1 and 2 are described in WO 2014/135709 A1, the contents of which are hereby incorporated by reference. In particular, it is described therein how such a watch module may be removably mounted to a wrist strap.

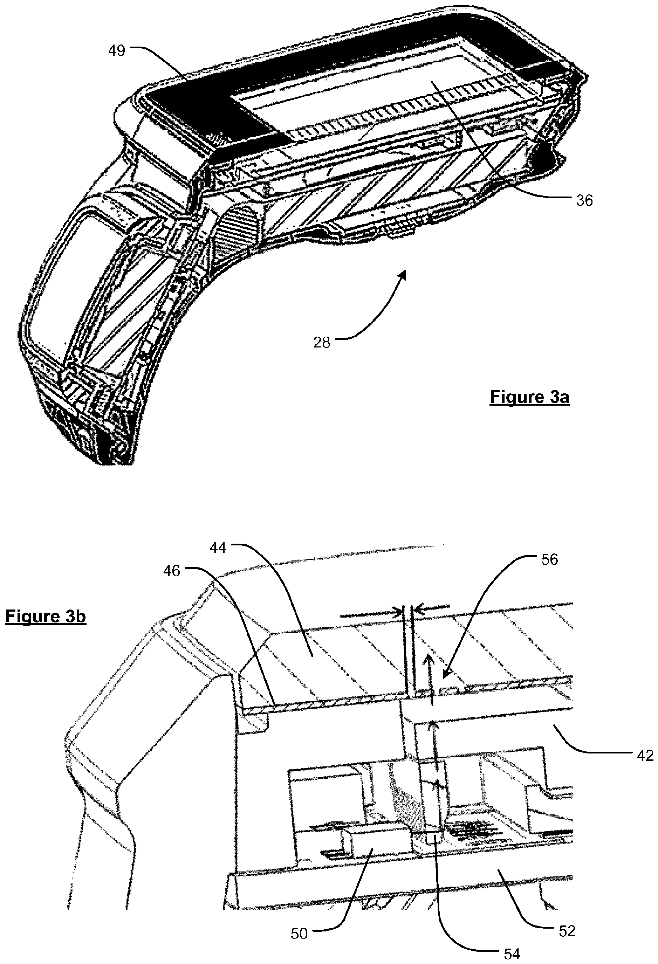

The display 36 seen in Figure 1 will now be described in more detail with reference to Figures 3a and 3b. The display 36 comprises a liquid crystal display (LCD) 42 mounted below a glass cover 44. The cover 44 includes on its underside a printed layer 46 that forms an opaque area 48 which hides the components below from view. The opaque area 48 defines a frame around the periphery of the display 36. In addition to the information displayed by the LCD 42, a LED 50, such as a tri-colour LED, is included in the watch module to convey heart rate information to a user, e.g. when the LCD 42 is not active. The LED 50 is mounted on the same printed circuit board 52 as the LCD 42. A transparent plastic light guide 54 is arranged to transmit light from the LED 50 to the underside of the LCD 42, so that its light passes through the LCD glass before reaching the printed layer 46. The printed layer 46 above the light guide 54 is interrupted by a pattern of 0.2 mm holes 56 which allows light to be transmitted through the opaque area 48 of the cover 44. The holes 56 can be seen in more detail in Figure 6. The LED 50 is hidden from view until lit because the inside of the watch module is dark and the holes 56 in the screen printed layer 46 are not noticeable until light is guided from the LED 50 up through the transparent light guide 54, LCD 42 and glass cover 44. In Figure 3b there are seen arrows that show the approximate light path.

The LED 50 is controlled by a processor in the watch module 28 to convey information relating to a user's heart rate. In one example, the LED 50 blinks at the approximate frequency of the heart rate, thereby providing for visualisation of the heart rate at a glance. The user's heart rate can be obtained from a heart rate monitor, such as the OHR sensing unit 39 or an external heart rate sensor. In embodiments of the watch module that do not comprise and/or are not connected to a heart rate monitor, then a heart rate can be estimated (or approximated) based on one or more measured parameters indicative of the user's current level of physical exertion. In addition, or alternatively, in another example the colour of the LED 50 represents a particular heart rate zone. The LED 50 may blink according to the following colour scheme:

| HEART RATE ZONE | COLOUR |
|---|---|
| 1. Recover | Turquoise |
| 2. Fat Burn | Blue |
| 3. Endure | Green |
| 4. Speed | Purple |
| 5. Sprint | Red |

The LED 50 can also be used to provide alerts to the user, in addition to, or alternatively from, audio, haptic or other visual alerts. Alerts could be provide by periodically flashing the LED 50 or by activating the LED 50 for a continuous period of time. For example, the LED 50 could flash a particular colour when a GPS connection has been established or lost. The flashing and/or colour of the LED 50 could also be used in interval or zone training, with the flashing of the LED in a particular colour indicating, for example, that the user should start and/or end an interval, that the user is above and/or below a desired training or heart rate zone, etc. The flashing and/or colour of the LED 50 could also be used when competing against a previous completed or simulated exercise or run, with the flashing of the LED in a particular colour indicating, for example, that the user is ahead and/or behind schedule.

It will be appreciated that whilst various aspects and embodiments of the present invention have heretofore been described, the scope of the present invention is not limited to the particular arrangements set out herein and instead extends to encompass all arrangements, and modifications and alterations thereto, which fall within the scope of the appended claims.

For example, whilst a preferred embodiment described in the foregoing detailed description relates to a watch module comprising a tri-colour LED, it will be appreciated that one or more LEDs may be employed, which emit one, two, three, four, five or more different colours. Similarly, whilst a preferred embodiment described in the foregoing detailed description relates to a watch module that can be removably mounted to a wrist strap, it will be understood that the module could be integrated with a wrist strap. Furthermore, although the watch module has been described as having an input device, this is an optional component. A suitable watch module may include a battery and a processor connected to one or more of: the display, an optional input device, a memory, a wireless transceiver, and an input/output device such as electrical contacts.

## Claims

1. A display for a watch case comprising: a display module; a substantially transparent cover extending over the display module, the cover comprising a layer (46) forming a substantially opaque area (48) outside the display module; and one or more light emitting devices (50) arranged below the opaque area, wherein the layer comprises a plurality of discontinuities that allow light to be transmitted through the opaque area from the one or more light emitting devices, wherein the plurality of discontinuities comprises holes (56) in the layer that are between 0.1 and 0.5 mm wide, such that the one or more light emitting devices are hidden from view when not in use.

2. The display of claim 1, wherein the opaque area forms a substantially opaque frame surrounding the display module.

3. The display of claim 1 or 2, wherein the layer is a printed layer.

4. The display of any preceding claim, wherein the holes in the layer are about 0.2 mm wide.

5. The display of any preceding claim, wherein the plurality of discontinuities are formed in a substantially uniform pattern.

6. The display of any preceding claim, further comprising at least one light guide arranged below the cover to guide light from the one or more light emitting devices to the plurality of discontinuities in the layer of the cover.

7. The display of any preceding claim, wherein the light is guided so as to pass through the display module before reaching the plurality of discontinuities in the layer of the cover.

8. The display of any preceding claim, wherein the one or more light emitting devices are arranged next to the display module and mounted on a shared circuit board.

9. The display of any preceding claim, wherein the one or more light emitting devices comprise LEDs.

10. The display of any preceding claim, wherein the one or more light emitting devices emit a plurality of different colours of light.

11. A watch case comprising the display of any preceding claim.

12. The watch case of claim 11, further comprising:
a transceiver and processor arranged to receive heart rate information from an external heart rate monitor;
an optical heart rate monitor arranged to measure heart rate information; or
a global navigation satellite system (GNSS) receiver and processor arranged to measure one or more parameters relating to a user's activity, wherein heart rate information is determined indirectly from the one or more parameters.

13. The watch case of any one of claims 11 to 12, wherein the one or more light emitting devices are controlled so as to flash at a frequency that is dependent on heart rate information.

14. The watch case of any one of claims 11 to 13, wherein the one or more light emitting devices are controlled so that the colour of emitted light corresponds to a predetermined heart rate zone.

15. A fitness watch comprising the watch case of any one of claims 11 to 14 and a strap for securing the watch to the arm or wrist of a user.

## Patentansprüche

1. Display für ein Uhrengehäuse, das Folgendes umfasst: ein Anzeigemodul; eine im Wesentlichen transparente Abdeckung, die sich über das Anzeigemodul erstreckt, wobei die Abdeckung eine Schicht (46) umfasst, die einen im Wesentlichen opaken Bereich (48) außerhalb des Anzeigemoduls bildet; und eine oder mehrere Licht emittierende Bauelemente (50), die unterhalb des opaken Bereichs angeordnet sind, wobei die Schicht mehrere Diskontinuitäten umfasst, die es zulassen, dass Licht durch den opaken Bereich von den ein oder mehreren Licht emittierenden Bauelementen durchgelassen wird, wobei die mehreren Diskontinuitäten Löcher (56) mit einer Breite zwischen 0,1 und 0,5 mm in der Schicht umfassen, so dass die ein oder mehreren Licht emittierenden Bauelemente nicht sichtbar sind, wenn sie nicht im Gebrauch sind.

2. Display nach Anspruch 1, wobei der opake Bereich einen im Wesentlichen opaken Rahmen um das Anzeigemodul herum bildet.

3. Display nach Anspruch 1 oder 2, wobei die Schicht eine gedruckte Schicht ist.

4. Display nach einem vorherigen Anspruch, wobei die Löcher in der Schicht eine Breite von etwa 0,2 mm haben.

5. Display nach einem vorherigen Anspruch, wobei die mehreren Diskontinuitäten in einem im Wesentlichen gleichförmigen Muster ausgebildet sind.

6. Display nach einem vorherigen Anspruch, das ferner wenigstens einen Lichtleiter umfasst, der unterhalb der Abdeckung angeordnet ist, um Licht von den ein oder mehreren Licht emittierenden Bauelementen zu den mehreren Diskontinuitäten in der Schicht der Abdeckung zu leiten.

7. Display nach einem vorherigen Anspruch, wobei das Licht so geleitet wird, dass es durch das Anzeigemodul passiert, bevor es die mehreren Diskontinuitäten in der Schicht der Abdeckung erreicht.

8. Display nach einem vorherigen Anspruch, wobei die ein oder mehreren Licht emittierenden Bauelementen neben dem Anzeigemodul angeordnet und auf einer gemeinsamen Leiterplatte montiert sind.

9. Display nach einem vorherigen Anspruch, wobei die ein oder mehreren Licht emittierenden Bauelemente LEDs umfassen.

10. Display nach einem vorherigen Anspruch, wobei die ein oder mehreren Licht emittierenden Bauelemente mehrere unterschiedliche Lichtfarben emittieren.

11. Uhrengehäuse, das das Display nach einem vorherigen Anspruch umfasst.

12. Uhrengehäuse nach Anspruch 11, das ferner Folgendes umfasst:
einen Transceiver und einen Prozessor, ausgelegt zum Empfangen von Herzfrequenzinformationen von einem externen Herzfrequenzmonitor;
einen optischen Herzfrequenzmonitor, ausgelegt zum Messen von Herzfrequenzinformationen; oder
einen GNSS-(Global Navigation Satellite System)-Empfänger und Prozessor, ausgelegt zum Messen von einem oder mehreren Parametern in Bezug auf eine Aktivität eines Benutzers, wobei Herzfrequenzinformationen indirekt von den ein oder mehreren Parametern bestimmt werden.

13. Uhrengehäuse nach einem der Ansprüche 11 bis 12, wobei die ein oder mehreren Licht emittierenden Bauelemente so gesteuert werden, dass sie mit einer Frequenz blinken, die von Herzfrequenzinformationen abhängig ist.

14. Uhrengehäuse nach einem der Ansprüche 11 bis 13, wobei die ein oder mehreren Licht emittierenden Bauelemente so gesteuert werden, dass die Farbe von emittiertem Licht einer vorbestimmten Herzfrequenzzone entspricht.

15. Fitnessuhr, die das Uhrengehäuse nach einem der Ansprüche 11 bis 14 und einen Riemen zum Befestigen der Uhr am Arm oder Handgelenk eines Benutzers umfasst.

## Revendications

1. Affichage pour un boîtier de montre comprenant : un module d'affichage ; un couvercle substantiellement transparent s'étendant par-dessus le module d'affichage, le couvercle comprenant une couche (46) formant une aire substantiellement opaque (48) à l'extérieur du module d'affichage ; et un ou plusieurs dispositifs électroluminescents (50) agencés en dessous de l'aire opaque, la couche comprenant une pluralité de discontinuités qui permettent à la lumière d'être transmise à travers l'aire opaque à partir desdits un ou plusieurs dispositifs électroluminescents, la pluralité de discontinuités comprenant des trous (56) dans la couche qui ont une largeur entre 0,1 et 0,5 mm, de telle sorte que lesdits un ou plusieurs dispositifs électroluminescents soient cachés de la vue lorsqu'ils ne sont pas en utilisation.

2. Affichage de la revendication 1, dans lequel l'aire opaque forme un cadre substantiellement opaque qui entoure le module d'affichage.

3. Affichage de la revendication 1 ou 2, dans lequel la couche est une couche imprimée.

4. Affichage de n'importe quelle revendication précédente, dans lequel les trous dans la couche ont une largeur d'environ 0,2 mm.

5. Affichage de n'importe quelle revendication précédente, dans lequel la pluralité de discontinuités sont formées en un schéma substantiellement uniforme.

6. Affichage de n'importe quelle revendication précédente, comprenant en outre au moins un guide de lumière agencé en dessous du couvercle afin de guider la lumière à partir desdits un ou plusieurs dispositifs électroluminescents jusqu'à la pluralité de discontinuités dans la couche du couvercle.

7. Affichage de n'importe quelle revendication précédente, dans lequel la lumière est guidée de sorte à passer à travers le module d'affichage avant d'atteindre la pluralité de discontinuités dans la couche du couvercle.

8. Affichage de n'importe quelle revendication précédente, dans lequel lesdits un ou plusieurs dispositifs électroluminescents sont agencés à côté du module d'affichage et sont montés sur une carte à circuits partagée.

9. Affichage de n'importe quelle revendication précédente, dans lequel lesdits un ou plusieurs dispositifs électroluminescents comprennent des DEL.

10. Affichage de n'importe quelle revendication précédente, dans lequel lesdits un ou plusieurs dispositifs électroluminescents émettent une pluralité de différentes couleurs de lumière.

11. Boîtier de montre comprenant l'affichage de n'importe quelle revendication précédente.

12. Boîtier de montre de la revendication 11, comprenant en outre :
un émetteur-récepteur et un processeur agencés pour recevoir des informations de rythme cardiaque à partir d'un moniteur externe de rythme cardiaque ;
un moniteur optique de rythme cardiaque agencé pour mesurer des informations de rythme cardiaque ; ou
un récepteur de système global de navigation par satellites (GNSS) et un processeur agencés pour mesurer un ou plusieurs paramètres relatifs à l'activité d'un utilisateur, des informations de rythme cardiaque étant déterminées indirectement à partir desdits un ou plusieurs paramètres.

13. Boîtier de montre de n'importe laquelle des revendications 11 à 12, dans lequel lesdits un ou plusieurs dispositifs électroluminescents sont commandés de sorte à clignoter à une fréquence qui dépend d'informations de rythme cardiaque.

14. Boîtier de montre de n'importe laquelle des revendications 11 à 13, dans lequel lesdits un ou plusieurs dispositifs électroluminescents sont commandés de sorte que la couleur de la lumière émise corresponde à une zone de rythme cardiaque prédéterminée.

15. Montre de sport comprenant le boîtier de montre de n'importe laquelle des revendications 11 à 14 et une sangle / un bracelet pour fixer la montre au bras ou au poignet d'un utilisateur.
